(19) Europäisches Patentamt
European Patent Office
Office européen des brevets

(11) Veröffentlichungsnummer: **0 008 649**
**B1**

(12) # EUROPÄISCHE PATENTSCHRIFT

(45) Veröffentlichungstag der Patentschrift: **27.01.82**

(21) Anmeldenummer: **79102528.1**

(22) Anmeldetag: **18.07.79**

(51) Int. Cl.³: **A 01 N 25/32,** A 01 N 43/56, A 01 N 43/64, C 07 C 103/34, C 07 D 207/02, C 07 D 231/02, C 07 D 233/00, C 07 D 249/02, C 07 D 257/02

(54) **Herbicide Mittel auf der Basis von Acetaniliden und N-Isopropyl-N-propargyl-dichloracetamid und Verfahren zur selektiven Bekämpfung von unerwünschtem Pflanzenwuchs.**

(30) Priorität: **27.07.78 DE 2832950**

(43) Veröffentlichungstag der Anmeldung: **19.03.80 Patentblatt 80/6**

(45) Bekanntmachung des Hinweises auf die Patenterteilung: **27.01.82 Patentblatt 82/4**

(84) Benannte Vertragsstaaten: **AT BE CH DE FR GB IT LU NL SE**

(56) Entgegenhaltungen:
**EP-A-0 001 751**
**FR-A-2 153 002**
**FR-A-2 215 170**
**FR-A-2 368 476**
**FR-A-2 379 525**
**NL-A-72 04 894**

(73) Patentinhaber: **BASF Aktiengesellschaft, Carl-Bosch-Strasse 38, D-6700 Ludwigshafen (DE)**

(72) Erfinder: **Hansen, Hanspeter, Dr. Chem., Thorwaldsenstrasse 5, D-6700 Ludwigshafen (DE)**
Erfinder: **Eicken, Karl, Dr. Chem., Waldstrasse 63, D-6706 Wachenheim (DE)**
Erfinder: **Plath, Peter, Dr. Chem., Berner Weg 24, D-6700 Ludwigshafen (DE)**
Erfinder: **Rohr, Wolfgang, Dr. Chem., Gontardstrasse 4, D-6800 Mannheim 1 (DE)**
Erfinder: **Wuerzer, Bruno, Dr. Dipl.-Landwirt, Wilhelm-Busch-Strasse 55, D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Herbizide Mittel auf der Basis von Acetaniliden und N-Isopropyl-N-propargyl-dichloracetamid und Verfahren zur selektiven Bekämpfung von unerwünschtem Pflanzenwuchs

Die vorliegende Anmeldung betrifft herbizide Mittel, die substituierte Acetanilide als herbizide Wirkstoffe und N-Isopropyl-N-propargyl-dichloracetamid als antagonistisches Mittel enthalten, sowie Verfahren zur selektiven Bekämpfung von unerwünschtem Pflanzenwuchs mit diesen herbiziden Mitteln.

Substituierte Acetanilide der Formel I

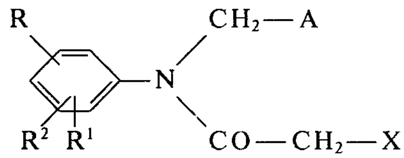

(I)

in der

R Wasserstoff, einen unverzweigten oder verzweigten Alkyl- oder Alkoxyrest mit bis zu 5 Kohlenstoffatomen,

$R^1$ Wasserstoff, Halogen, einen unverzweigten oder verzweigten Alkyl- oder Alkoxyrest mit bis zu 5 Kohlenstoffatomen,

$R^2$ Wasserstoff, Halogen, einen unverzweigten oder verzweigten Alkyl- oder Alkoxyrest mit bis zu 5 Kohlenstoffatomen,

R zusammen mit $R^2$ eine orthoständig verknüpfte, gegebenenfalls durch unverzweigte oder verzweigte Alkylgruppen mit bis zu 4 Kohlenstoffatomen substituierte Alkylenkette mit bis zu 6 Kohlenstoffatomen,

X Chlor oder Brom und

A ein über ein Ringstickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy, Carbalkoxy mit bis zu 4 Kohlenstoffatomen in der Alkoxygruppe oder Alkanoylreste mit bis zu 4 Kohlenstoffatomen substituiert sein kann, bedeutet, wobei A auch für Salze der 2 oder 3 Stickstoffatome enthaltenden Azole stehen kann,

sind herbizid ausgezeichnet wirksam, führen aber bei Anwendung in Kulturen, wie Mais, oder in Kulturen aus der Familie der Gramineen zu Schädigungen der Nutzpflanzen.

Aufgabe der Erfindung war es deshalb, antagonistische Mittel zu finden, die diese Unverträglichkeit der herbiziden Acetanilide gegenüber bestimmten Kulturpflanzen kompensieren.

Antagonistische Mittel (Antidots) sind chemische Verbindungen, durch deren Präsenz eine Steigerung der Verträglichkeit von für bestimmte Kulturpflanzen nicht oder nicht ausreichend selektiven herbiziden Wirkstoffen erreicht wird. Die Aktivität der herbiziden Wirkstoffe gegen unerwünschte Pflanzen bleibt dabei unbeeinflußt.

Herbizide Mittel, die neben Chloracetaniliden als herbiziden Wirkstoffen antagonistisch wirkende Dichloracetamide enthalten, sind aus der DE-OS 2 218 097, der DE-OS 2 402 983 und der US-PS 4 053 297 bekannt.

In der DE-OS 2 218 097 werden beispielsweise Kombinationen aus antagonistisch wirkendem N,N-Diallyl-dichloracetamid und herbiziden Wirkstoffen aus der Klasse der Thiolcarbamate, der Triazine oder der Acetanilide, wie 2-Chlor-2',6'-diäthyl-N-methoxymethyl-acetanilid, erwähnt. Dieses Dichloracetamid zeigt allerdings in Kombination mit herbiziden Acetaniliden eine schwache antagonistische Wirkung.

Gegenstand der DE-OS 2 402 983 und der US-PS 4 053 297 sind herbizide Mittel, die aus der DE-OS 2 218 097 bereits bekannte oder diesen strukturell ähnliche Dichloracetamide zusammen mit Chloracetaniliden anderer Konstitution enthalten. Diese Mittel eignen sich zur selektiven Unkrautbekämpfung in Mais.

Es wurde gefunden, daß N-Isopropyl-N-propargyl-dichloracetamid sich ausgezeichnet zur Steigerung der Kulturpflanzenverträglichkeit von herbiziden substituierten Acetaniliden der Formel I eignet. Herbizide Mittel, die mindestens ein substituiertes Acetanilid der Formel I und N-Isopropyl-N-propargyl-dichloracetamid enthalten, können sowohl in Mais als auch in Getreidekulturen eingesetzt werden. Dabei bleibt die gute herbizide Wirkung der Acetanilide erhalten, während Schädigungen an den Nutzpflanzen unterbunden werden.

Acetanilide, deren Kulturpflanzenverträglichkeit durch das Dichloracetamid verbessert wird, sind solche, bei denen

R für Wasserstoff, Alkyl mit bis zu 5 Kohlenstoffatomen, wie Methyl, Äthyl, n-Propyl, i-Propyl, n-Butyl, sec.-Butyl, i-Butyl, tert.-Butyl, normale und verzweigte Pentylreste, Alkoxy mit bis zu 5 Kohlenstoffatomen, wie Methoxy, Äthoxy, Propoxy, Butoxy, Pentyloxy;

$R^1$ und $R^2$ für Wasserstoff, Halogen, wie Fluor, Chlor, Brom oder Jod, Alkyl bis zu 5 Kohlenstoffatomen, wie Methyl, Äthyl, n-Propyl, i-Propyl, n-Butyl, sec.-Butyl, i-Butyl, tert.-Butyl, n-Pentyl und verzweigte Pentylreste, Alkoxy mit bis zu 5 Kohlenstoffatomen, wie Methoxy, Äthoxy, Propoxy, Butoxy, Pentyloxy;

R zusammen $R^2$ für eine orthoständig verknüpfte, gegebenenfalls durch Alkyl mit bis zu 4 Kohlenstoffatomen substituierte Alkylenkette mit bis zu 6 Kohlenstoffatomen, wie Äthylen, Trimethylen, Tetramethylen, 1-Methyl-trimethylen, 1,1-Dimethyl-trimethylen, 1,1-Dimethyl-tetramethylen;

X für Chlor oder Brom, vorzugsweise für Chlor, und

A für ein über ein Ring-Stickstoffatom gebundenes Azol, wie Pyrrol, Pyrazol, Imidazol, 1,2,4-Triazol, 1,2,3-Triazol, Tetrazol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy, Carbalkoxyreste mit bis zu 4 Kohlenstoffatomen in der Alkoxygruppe oder Alkanoyl mit bis zu 4 Kohlenstoffatomen unabhängig voneinander substituiert sein kann, wie

2,6-Dimethylpyrrol, Tetramethylpyrrol, 3(5)-Methylpyrazol, 4-Methylpyrazol, 3(5)-Äthylpyrazol, 4-Äthylpyrazol, 3(5)-Isopropylpyrazol, 4-Isopropylpyrazol, 3,5-Dimethylpyrazol, 3,5-Dimethyl-4-acetylpyrazol, 3,5-Dimethyl-4-propionylpyrazol, 3,4,5-Trimethylpyrazol, 3(5)-Phenylpyrazol, 4-Phenylpyrazol, 3,5-Diphenylpyrazol, 3(5)-Phenyl-5(3)-methylpyrazol, 3(5)-Chlorpyrazol, 4-Chlorpyrazol, 4-Brompyrazol, 4-Jodpyrazol, 3,4,5-Trichlorpyrazol, 3,4,5-Tribrompyrazol, 3,5-Dimethyl-4-chlorpyrazol, 3,5-Dimethyl-4-brompyrazol, 4-Chlor-(3(5)-methylpyrazol, 4-Brom-3(5)-methylpyrazol, 4-Methyl-3,5-dichlorpyrazol, 3(5)-Methyl-4,5(3)-dichlorpyrazol, 3(5)-Chlor-5(3)-methylpyrazol, 4-Methoxypyrazol, 3(5)-Methyl-5(3)-methoxypyrazol, 3(5)-Äthoxy-4,5(3)-dimethylpyrazol, 3(5)-Methyl-5(3)-trifluormethyl-pyrazol, 3,5-Bistrifluormethylpyrazol, 3(5)-Methyl-5(3)-carbäthoxy-pyrazol, 3,5-Biscarbäthoxypyrazol, 3,4,5-Triscarbäthoxypyrazol, 3(5)-Methyl-5(3)-methylthio-4-carbäthoxypyrazol, 4-Methyl-3,5-biscarbäthoxypyrazol, 4-Cyanopyrazol, 4-Methoxy-3,5-dichlorpyrazol, 4,5-Dichlor-imidazol, 2-Äthyl-4,5-dichlor-imidazol, 2-Methyl-4,5-dichlorimidazol, 3(5)-Methyl-1,2,4-triazol, 3,5-Dimethyl-1,2,4-triazol, 3(5)-Chlor-1,2,4-triazol, 3(5)-Brom-1,2,4-triazol, 3(5)-Chlor-5(3)-methyl-1,2,4-triazol, 3,5-Dichlor-1,2,4-triazol, 3,5-Dibrom-1,2,4-triazol, 3(5)-Chlor-5(3)-cyano-1,2,4-triazol, 3(5)-Chlor-5(3)-phenyl-1,2,4-triazol, 3(5)-Chlor-5(3)-carbomethoxy-1,2,4-triazol, 3(5)-Methylthio-1,2,4-triazol, 4(5)-Methyl-1,2,3-triazol, 4,5-Dimethyl-1,2,3-triazol, 4(5)-Phenyl-1,2,3-triazol, 4(5)-Chlor-1,2,3-triazol, 1,2,3-Triazol-4(5)-yl-carbonsäureäthylester, 1,2,3-Triazol-4,5-yl-dicarbonsäuredimethylester, 5-Methyltetrazol, 5-Chlortetrazol, Tetrazolyl-5-carbonsäureäthylester, stehen.

Darüber hinaus kann der Rest A, wenn das gegebenenfalls substituierte Azol 2 oder 3 Stickstoffatome enthält, auch salzartig an eine der üblichen starken anorganischen oder organischen Säuren, wie Chlorwasserstoffsäure, Bromwasserstoffsäure, Salpetersäure, Schwefelsäure, Tetrafluorborsäure, Fluorsulfonsäure, Ameisensäure, eine halogenierte Carbonsäure, z. B. Trichloressigsäure, eine Alkansulfonsäure, z. B. Methansulfonsäure, eine halogenierte Alkansulfonsäure, z. B. Trifluormethansulfonsäure, Perfluorhexansulfonsäure, eine Arylsulfonsäure, z. B. Dodecylbenzolsulfonsäure, gebunden sein.

Bevorzugt sind Acetanilide, die in 2- und 6-Stellung am Phenylring Methyl oder Äthyl und in 3-Stellung Wasserstoff, Methyl oder Äthyl tragen, wobei als Azole Pyrazol, Imidazol, Triazol oder Tetrazol, die jeweils durch niederes Alkyl, Alkoxy, Carbalkoxy, Cyan oder Halogen substituiert sein können, in Betracht kommen.

Insbesondere enthalten die erfindungsgemäßen herbiziden Mittel folgende Acetanilide:

2-Chlor-2',6'-dimethyl-N-(pyrazol-1-yl-methyl)-acetanilid,
2-Chlor-2'-methyl-6'-äthyl-N-(pyrazol-1-yl-methyl)-acetanilid,
2-Chlor-2',6'-dimethyl-N-(4-methylpyrazol-1-yl-methyl)-acetanilid,
2-Chlor-2'-methyl-6'-äthyl-N-(4-methoxypyrazol-1-yl-methyl)-acetanilid,
2-Chlor-2'-methyl-6'-äthyl-N-(3-(5)-methylpyrazol-1-yl)-acetanilid,
2-Chlor-2',6'-dimethyl-N-(3,5-dimethylpyrazol-1-yl-methyl)-acetanilid,
2-Chlor-2',6'-dimethyl-N-(1,2,4-triazol-1-yl-methyl)-acetanilid,
2-Chlor-2',6'-dimethyl-N-(4-chlorpyrazol-1-yl-methyl)-acetanilid,
2-Chlor-2',3',6'-trimethyl-N-(pyrazol-1-yl-methyl)-acetanilid,
2-Chlor-2'-methyl-6'-äthyl-N-(3,5-dimethyl-pyrazol-1-yl-methyl)-acetanilid,
2-Chlor-2',6'-diäthyl-N-(3,5-dimethyl-pyrazol-1-yl-methyl)-acetanilid,

2-Chlor-2',3',6'-trimethyl-N-(3,5-dimethyl-pyrazol-1-yl-methyl)-acetanilid,
2-Chlor-2',6'-diäthyl-N-(4-methylpyrazol-1-yl-methyl)-acetanilid,
2-Chlor-2'-methyl-6'-äthyl-N-(4-methylpyrazol-1-yl-methyl)-acetanilid,
2-Chlor-2',3',6'-trimethyl-N-(4-methylpyrazol-1-yl-methyl)-acetanilid,
2-Chlor-2',6'-dimethyl-N-(3-(5)-methylpyrazol-1-yl-methyl)-acetanilid,
2-Chlor-2',6'-diäthyl-N-(3-(5)-methylpyrazol-1-yl-methyl)-acetanilid,
2-Chlor-2',6'-dimethyl-N-(4-methoxypyrazol-1-yl-methyl)-acetanilid,
2-Chlor-2',6'-diäthyl-N-(pyrazol-1-yl-methyl)-acetanilid,
2-Chlor-2',6'-dimethyl-N-(4,5-dichlorimidazol-1-yl-methyl)-acetanilid,
2-Chlor-2'-methyl-6'-äthyl-N-(4,5-dichlorimidazol-1-yl-methyl)-acetanilid,
2-Chlor-2'-methyl-6'-äthyl-N-(2-äthyl-4,5-dichlorimidazol-1-yl-methyl)-acetanilid,
2-Chlor-2',6'-diäthyl-N-(4,5-dichlorimidazol-1-yl-methyl)-acetanilid,
2-Chlor-2'-methyl-6'-äthyl-N-(1,2,4-triazol-1-yl-methyl)-acetanilid,
2-Chlor-2',6'-diäthyl-N-(1,2,4-triazol-1-yl-methyl)-acetanilid und
2-Chlor-2',3',6'-trimethyl-N-(1,2,4-triazol-1-yl-methyl)-acetanilid.

Die Acetanilide der Formel I sind Gegenstand der DE-OS 2 648 008 und der DE-OS 2 744 396. Sie können durch Umsetzung von 2-Halogen-N-halogenmethylacetaniliden der Formel III mit einem 1 H-Azol der Formel H-A nach folgender Reaktionsgleichung erhalten werden:

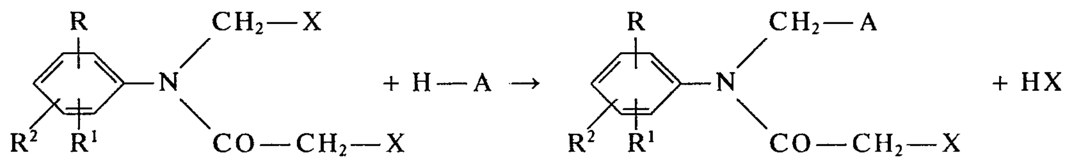

(III) (I)

Dabei haben die Substituenten, R, $R^1$, $R^2$ und X die oben genannte Bedeutung und A steht für ein über ein Ring-Stickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy, Carbalkoxyreste mit bis zu 4 Kohlenstoffatomen in der Alkoxygruppe oder Alkanoylreste mit bis zu 4 Kohlenstoffatomen substituiert sein kann.

N-Isopropyl-N-propargyl-dichloracetamid kann durch Umsetzung von N-Isopropyl-N-propargyl-amin mit Dichloracetylchlorid erhalten werden. Die Umsetzung wird in an sich bekannter Weise in Gegenwart eines Chlorwasserstoff-bindenden Mittels in einem inerten Lösungs- oder Verdünnungsmittel durchgeführt.

Chlorwasserstoff-bindende Mittel können anorganische Basen, wie Alkalicarbonate, Alkalihydrogencarbonate, Alkalihydroxide, oder organische Basen, beispielsweise tertiäre Amine, wie Trialkylamine, insbesondere Triäthylamin, sein.

Als inerte Lösungs- oder Verdünnungsmittel kommen Kohlenwasserstoffe, wie Toluol, Cyclohexan, halogenierte Kohlenwasserstoffe, wie Methylenchlorid, Äthylenchlorid, und Äther, wie Dioxan, Tetrahydrofuran, in Betracht.

Das folgende Beispiel erläutert die Herstellung des Dichloracetamids.

## Beispiel

118 g Isopropyl-propargyl-amin und 123 g Triäthylamin werden in 670 ml Toluol gelöst. Zu dieser Lösung tropft man unter Kühlung bei Raumtemperatur 180 g Dichloracetylchlorid, gelöst in 450 ml Toluol, zu. Man läßt eine Stunde lang nachreagieren, saugt ab und wäscht das Filtrat mit Wasser. Der nach dem Abziehen des Lösungsmittels verbleibende Rückstand wird mit Petroläther gewaschen. Man erhält 214 g N-Isopropyl-N-propargyl-dichloracetamid, entsprechend einer Ausbeute von 84% d. Th.; Kp.: 80—82° C/0,013 mbar; Schmp.: 58—59° C.

Das antagonistisch wirkende Dichloracetamid hat selbst keinen oder kaum sichtbaren Einfluß auf Keimung und Wachstum sowohl von Kulturpflanzen als auch von unerwünschten Pflanzen, selbst bei Aufwandmengen, welche beträchtlich über denjenigen liegen, welche zur Erzielung eines antagonistischen Effektes benötigt werden. Es hat jedoch die Fähigkeit, die Phytotoxizität der herbiziden Acetanilide der Formel I gegenüber Kulturpflanzen, wie Mais, beachtlich zu reduzieren oder völlig zu eliminieren.

Für herbizide Acetanilide, die gegenüber den Kulturpflanzen weniger aggressiv sind, genügen geringere Zusätze an antagonistisch wirkender Verbindung. Das Anteilsverhältnis Acetanilid : Dichloracetamid kann in Abhängigkeit vom Acetanilid stark schwanken. Geeignete Anteilsverhältnisse herbizider Wirkstoff : antagonistisch wirkender Verbindung liegen zwischen 1 : 2 bis 1 : 0,05 Gewichtsteilen.

Acetanilide und Dichloracetamid können gemeinsam oder getrennt, vor oder nach der Saat, in den Boden eingearbeitet werden. Für die Acetanilide der Formel I ist das gebräuchlichste Anwendungsverfahren die Ausbringung auf die Erdoberfläche unmittelbar nach Ablage der Samen oder in der Zeit zwischen Einsaat und Auflaufen der jungen Pflanzen. Auch eine Behandlung während des Auflaufens und kurz danach ist möglich. In jedem Fall kann die antagonistisch wirkende Verbindung gleichzeitig mit dem herbiziden Wirkstoff ausgebracht werden. Auch eine getrennte Ausbringung, wobei der Antagonist zuerst und anschließend das Herbizid oder umgekehrt auf das Feld gebracht werden, ist möglich, sofern im letzteren Fall nicht so viel Zeit verstreicht, daß das Herbizid bereits Schaden an den Kulturpflanzen angerichtet hat. Herbizid und Antagonist können hierbei als Spritzmittel in suspendierbarer, emulgierbarer oder löslicher Form oder als Granulate, getrennt oder gemeinsam formuliert, vorliegen. Denkbar ist auch eine Behandlung der Kulturpflanzensamen mit dem Antagonisten vor der Aussaat. Das Herbizid wird dann allein in der üblichen Weise appliziert.

Die neuen herbiziden Mittel können neben Acetanilid und Dichloracetamid weitere herbizide oder wachstumsregulierende Wirkstoffe anderer chemischer Struktur, beispielsweise 2-Chlor-4-äthylamino-6-isopropyl-amino-1,3,5-triazin, enthalten, ohne daß der antagonistische Effekt des Dichloracetamids beeinflußt wird.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentige wäßrige, ölige oder sonstige Suspensionen, oder Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsformen richten sich ganz nach den Verwendungszwecken; sie sollten in jedem Fall möglichst die feinste Verteilung der erfindungsgemäßen Mittel bzw. der Einzelkomponenten gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Äthanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel in Wasser homogenisiert werden. Es können aber auch aus herbizidem Wirkstoff und/oder Antidot, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze oder Dibutylnaphthalinsäulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatierten Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol, Octylphenol, Nonylphenol, Alkylphenolpolyglykoläther, Tributylphenylpolyglykoläther, Alkalarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolylglykolätheracetal, Sorbitester, Lignin, Sulfitablaugen und Methylcellulose in Betracht.

Pulver, Streu- und Stäubemittel können durch Mischen oder gemeinsames Vermahlen von herbizidem Wirkstoff und/oder Antidot mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Treägerstoffe.

Die Formulierungen enthalten zwischen 0,1 und 95 Gewichtsprozent an herbizidem Wirkstoff und/oder Antidot, vorzugsweise zwischen 0,5 und 90 Gewichtsprozent. Die Aufwandmengen an herbizidem Wirkstoff betragen 0,2 bis 5 kg/ha. Diese Menge an herbizidem Wirkstoff wird, gemeinsam oder getrennt, mit einer solchen Menge an Antidot ausgebracht, daß das Anteilsverhältnis herbizider Wirkstoff : antagonistischer Verbindung 1 : 2 bis 1 : 0,05 Gewichtsteile beträgt.

Ein Beispiel für die Herstellung einer Formulierung ist: 15 Gewichtsteile der Mischung aus einem

Gewichtsteil 2-Chlor-2'-methyl-6'-äthyl-N-(1,2,4-triazol-1-yl-methyl)-acetanilid und einem Gewichtsteil N-Propargyl-N-isopropyl-dichloracetamid werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Gewächshaus- und Freilandversuche zeigen, daß N-Isopropyl-N-propargyl-dichloracetamid die Verträglichkeit der herbiziden Acetanilide für die Kulturpflanzen steigert, ohne ihre herbizide Aktivität zu reduzieren.

## I Gewächshausversuche

Plastikpikierkisten von 51 cm Länge, 32 cm Breite und 6 cm Höhe wurden mit lehmigem Sand von pH 6 und etwa 1,5% Humus gefüllt. In dieses Substrat wurde Mais (Zea mays) als Kulturpflanze in Reihen flach eingesät. Außerdem wurden Hühnerhirse und Ackerfuchsschwanz als unerwünschte Pflanzen breitwürfig hinzugegeben. Der nicht sterilisierte Boden enthielt zusätzlich lebensfähige Unkrautsamen, welche zur Population unerwünschter Pflanzen beitrugen. Dadurch wurde ein mit Kulturpflanzen bestellter und mit Unkräutern verseuchter Acker simuliert.

Die Wirkstoffe und die antagonistisch wirkende Verbindung wurden sowohl einzeln als auch in den beschriebenen Mischungen ausgebracht. Sie wurden, emulgiert oder suspendiert in Wasser als Trägermedium, mittels fein verteilender Düsen unmittelbar nach der Saat und vor Auflaufen der Testpflanzen auf die Erdoberfläche gespritzt. In einigen Fällen wurden die Mittel auch von Einsaat der Kulturpflanzen in den Boden eingemischt. Nach Einsaat und Behandlung werden die Kisten beregnet und bis nach dem Auflaufen der Pflanzen mit durchsichtigen Plastikhauben abgedeckt. Durch diese Maßnahme wurde ein gleichmäßiges Keimen und Anwachsen der Pflanzen garantiert. Die Aufstellung erfolgte in einem Temperaturbereich von durchschnittlich 18 bis 30° C.

Die so angelegten Gewächshausversuche wurden beobachtet, bis der Mais 3 bis 5 Blätter entwickelt hatte. Nach diesem Stadium waren bei den herbiziden Mitteln keine Schädigungen mehr zu erwarten, was auch in den Freilandversuchen bestätigt wurde.

Die Mitteleinwirkung wurde nach einer Skala von 0 bis 100 bewertet. Hierbei bedeutet 0 normaler Aufgang und normale Entwicklung der Pflanzen, bezogen auf die unbehandelte Kontrolle. 100 entspricht einem völligen Ausbleiben der Keimung bzw. Absterben der Pflanzen. Es mußte dabei berücksichtigt werden, daß beispielsweise bei Mais auch unter völlig normalen Verhältnissen und ohne jede chemische Behandlung vereinzelt verkrüppelte oder gehemmte Pflanzen vorkommen.

## II Freilandversuche

Die Freilandversuche wurden auf Kleinparzellen auf Standorten mit lehmigem Sand und Lehm vom pH 5 bis 6 und 1 bis 1,5% Humusgehalt angelegt. Die Vorauflaufbehandlungen erfolgten jeweils unmittelbar bis spätestens 3 Tage nach der Saat der Kulturpflanzen. Die Unkrautflora verschiedenster Artenzusammensetzung war natürlich. Allerdings wurden nur die dominierenden Vertreter in die Tabellen aufgenommen. Herbizide und Antagonist sowie ihre Kombinationen wurden in Wasser als Träger- und Verteilermedium emulgiert oder suspendiert und mittels einer motorgetriebenen, auf einen Traktor montierten Parzellenspritze ausgebracht. Bei Mangel an natürlichen Niederschlägen wurde zusätzlich beregnet, um ein normales Auflaufen von Kultur und Unkräutern zu gewährleisten. Alle Versuche liefen über mehrere Monate, so daß die Entwicklung der Kulturpflanze bis zur Samenbildung beobachtet werden konnte. Die Bewertung der Mittelwirkung wurde ebenfalls nach der Skala von 0 bis 100 vorgenommen.

## Ergebnis

Die im einzelnen in den folgenden Tabellen aufgeführten Versuchsergebnisse zeigen, daß das antagonistisch wirkende N-Isopropyl-N-propargyl-dichloracetamid bei seiner Anwendung in Kombination mit den herbiziden Chloracetaniliden der Formel I die Unverträglichkeit dieser Herbizide gegenüber den Kulturpflanzen kompensiert.

Bedingt durch die flache Einsaat der Kulturpflanzen und die für die herbizide Aktivität günstigeren Bedingungen traten die durch die Herbizide verursachten Schäden im Gewächshaus viel stärker zutage als im Freiland. Die Prüfung der antagonistisch wirkenden Verbindung war folglich im Gewächshaus härter als im Freiland.

Tabelle 1

Liste der Pflanzennamen

| Botanischer Name | Deutsche Bezeichnung | Englische Bezeichnung |
|---|---|---|
| Alopecurus myosuroides | Ackerfuchsschwanz | slender foxtail |
| Chenopodium album | Weißer Gänsefuß | lambsquarters |
| Echinochloa crus galli | Hühnerhirse | barnyardgrass |
| Zea mays | Mais | Indian corn |

Tabelle 2

Liste der in den biologischen Beispielen aufgeführten herbiziden Acetanilide

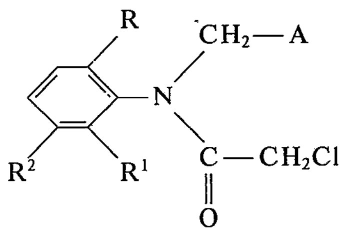

| Bezeichnung | A | R | $R^1$ | $R^2$ |
|---|---|---|---|---|
| I | 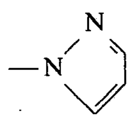 | $CH_3$ | $CH_3$ | H |
| II | 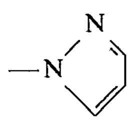 | $C_2H_5$ | $CH_3$ | H |
| III | 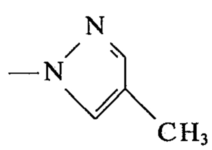 | $CH_3$ | $CH_3$ | H |
| IV | 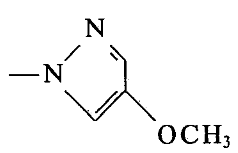 | $C_2H_5$ | $CH_3$ | H |
| V | 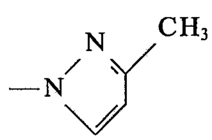 | $C_2H_5$ | $CH_3$ | H |
| VI | 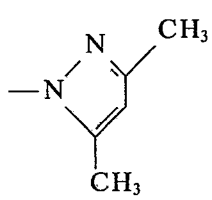 | $CH_3$ | $CH_3$ | H |

Fortsetzung

| Bezeichnung | A | R | $R^1$ | $R^2$ |
| --- | --- | --- | --- | --- |
| VII | 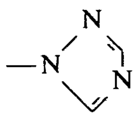 | $CH_3$ | $CH_3$ | H |
| VIII | 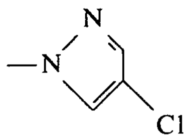 | $CH_3$ | $CH_3$ | H |
| IX | 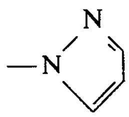 | $CH_3$ | $CH_3$ | $CH_3$ |

Tabelle 3

Liste der in den biologischen Beispielen aufgeführten Dichloracetamide

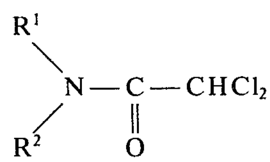

| Bezeichnung | $R^1$ | $R^2$ |
| --- | --- | --- |
| A | $-CH_2-CH=CH_2$ | $-CH_2CH=CH_2$ (bekannt aus DE-OS 2 218 097) |
| B | $-CH_2-C\equiv CH$ | $i\text{-}C_3H_7$ |

Tabelle 4

Verbesserung der Mais-Verträglichkeit von 2-Chlor-2',6'-dimethyl-N-(pyrazol-1-yl-methyl)-acetanilid durch N-Isopropyl-N-propargyl-dichloracetamid bei Vorauflaufanwendung im Gewächshaus

| Herbizider Wirkstoff | Antagonistisch wirkende Verbindungen | Aufwandmenge [kg/ha a.S.] | Testpflanzen und % Schädigung Kulturpflanze Zea mays | unerwünschte Pflanze Echinochloa crus galli |
|---|---|---|---|---|
| I | – | 1,0 | 63 | 100 |
| | | 2,0 | 78 | 100 |
| I | A | 2,0 | 4 | 8 |
| | + A | 1,0 + 0,125 | 34 | 100 |
| | | 1,0 + 0,25 | 22 | 99 |
| | | 2,0 + 0,5 | 43 | 100 |
| I | B | 2,0 | 3 | 0 |
| | + B | 1,0 + 0,125 | 17 | 98 |
| | | 1,0 + 0,25 | 13 | 97 |
| | | 1,0 + 0,5 | 7 | 96 |
| | | 1,0 + 1,0 | 12 | 100 |
| | | 2,0 + 0,5 | 23 | 100 |

Tabelle 5

Verbesserung der Kulturpflanzenverträglichkeit von herbiziden Acetaniliden bei Vorauflaufanwendung im Gewächshaus durch N-Isopropyl-N-propargyl-dichloracetamid

| Herbizide Wirkstoffe | Antagonistisch wirkende Verbindung | Aufwandmenge [kg/ha a.S.] | Testpflanzen und % Schädigung<br>Kulturpflanze<br>Zea mays | unerwünschte Pflanzen<br>Alopecurus myosuroides | <br>Echinochloa crus galli |
|---|---|---|---|---|---|
| II | – | 1,0 | 50 | 95 | 99 |
| | | 2,0 | 63 | 96 | 98 |
| II | + B | 1,0 + 0,125 | 5 | 98 | 98 |
| | | 1,0 + 0,25 | 3 | 96 | 98 |
| | | 1,0 + 2,0 | 5 | 85 | 98 |
| | | 2,0 + 0,5 | 5 | 98 | 99 |
| III | – | 1,0 | 30 | 88 | 99 |
| | | 2,0 | 47 | 99 | 99 |
| III | + B | 1,0 + 0,125 | 2 | 80 | 99 |
| | | 1,0 + 0,25 | 2 | 88 | 99 |
| | | 1,0 + 1,0 | 7 | 84 | 99 |
| | | 2,0 + 0,5 | 3 | 100 | 100 |
| IV | – | 1,0 | 17 | 90 | 99 |
| | | 2,0 | 42 | 90 | 99 |
| IV | + B | 1,0 + 0,125 | 2 | 89 | 99 |
| | | 1,0 + 1,0 | 5 | 88 | 99 |
| | | 2,0 + 0,5 | 6 | 92 | 100 |
| V | – | 1,0 | 60 | 90 | 99 |
| | | 2,0 | 65 | 94 | 99 |
| V | + B | 1,0 + 0,25 | 7 | 90 | 99 |
| | | 1,0 + 1,0 | 5 | 92 | 99 |
| | | 2,0 + 0,5 | 7 | 98 | 99 |
| VI | – | 1,0 | 73 | 98 | 99 |
| | | 2,0 | 85 | 98 | 99 |

Fortsetzung

| Herbizide Wirkstoffe | Antagonistisch wirkende Verbindung | Aufwandmenge [kg/ha a.S.] | Testpflanzen und % Schädigung Kulturpflanze Zea mays | unerwünschte Pflanzen Alopecurus myosuroides | Echinochloa crus galli |
|---|---|---|---|---|---|
| VI | + B | 1,0 + 1,0 | 18 | 93 | 98 |
| VII | – | 1,0 | 35 | 94 | 99 |
| | | 2,0 | 55 | 98 | 99 |
| VII | + B | 1,0 + 1,0 | 2 | 84 | 99 |
| VIII | – | 1,0 | 12 | 85 | 98 |
| | | 2,0 | 25 | 90 | 99 |
| VIII | + B | 1,0 + 0,125 | 5 | 80 | 98 |
| | | 2,0 + 0,25 | 2 | 96 | 98 |
| IX | – | 1,0 | 80 | 90 | 99 |
| | | 2,0 | 82 | 95 | 100 |
| IX | + B | 1,0 + 0,125 | 5 | 98 | 98 |
| | | 1,0 + 1,0 | 2,5 | 98 | 98 |
| | | 2,0 + 0,5 | 5 | 99 | 98 |

0 = keine Schädigung, 100 = Pflanzen nicht aufgelaufen oder abgestorben.

Tabelle 6

Verbesserung der Verträglichkeit von 2-Chlor-2', 6'-dimethyl-N-(pyrazol-1-yl-methyl)-acetanilid für Mais durch N-Isopropyl-N-propargyl-dichloracetamid bei Vorauflaufanwendung im Freiland

| Herbizider Wirkstoff | Antagonistisch wirkende Verbindung | Aufwandmenge [kg/ha a.S.] | Testpflanzen und % Schädigung Kulturpflanze Zea mays | unerwünschte Pflanzen Echinochloa cruss galli | Chenopodium album |
|---|---|---|---|---|---|
| I | – | 2,0 | 37 | 100 | 99 |
| | | 3,0 | 39 | 100 | 100 |
| | | 4,0 | 55 | 100 | 100 |
| | A | 4,0 | 0 | 0 | 0 |
| I | + A | 2,0 + 0,5 | 19 | 100 | 100 |
| | | 2,0 + 1,0 | 10 | 100 | 100 |
| | | 3,0 + 1,0 | 25 | 100 | 100 |
| | | 4,0 + 1,0 | 42 | – | – |
| | B | 4,0 | 0 | 0 | 0 |
| I | + B | 2,0 + 0,5 | 7 | 100 | 100 |
| | | 2,0 + 1,0 | 6 | 100 | 100 |
| | | 3,0 + 1,0 | 18 | 100 | 100 |
| | | 4,0 + 1,0 | 18 | 100 | 100 |

0 = normaler Aufgang und Wuchs, 100 = Pflanzen nicht aufgelaufen oder abgestorben.

**Patentansprüche**

1. Herbizides Mittel, gekennzeichnet durch einen Gehalt an mindestens einem substituierten Acetanilid der Formel I

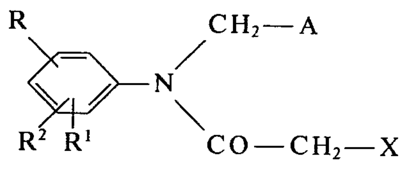

(I)

in der

R Wasserstoff, einen unverzweigten oder verzweigten Alkyl- oder Alkoxyrest mit bis zu 5 Kohlenstoffatomen,

$R^1$ Wasserstoff, Halogen, einen unverzweigten oder verzweigten Alkyl- oder Alkoxyrest mit bis zu 5 Kohlenstoffatomen,

$R^2$ Wasserstoff, Halogen, einen unverzweigten oder verzweigten Alkyl- oder Alkoxyrest mit bis zu 5 Kohlenstoffatomen,

R zusammen mit $R^2$ eine orthoständig verknüpfte, gegebenenfalls durch unverzweigte oder verzweigte Alkylgruppen mit bis zu 4 Kohlenstoffatomen substituierte Alkylenkette mit bis zu 6 Kohlenstoffatomen,

X Chlor oder Brom und

A ein über ein Ringstickstoffatom gebundenes Azol, das einfach oder mehrfach durch Halogen, Phenyl, Alkyl-, Alkoxy-, Alkylthio- oder Perfluoralkylreste mit jeweils bis zu 4 Kohlenstoffatomen, Cyan, Carboxy, Carbalkoxy mit bis zu 4 Kohlenstoffatomen in der Alkoxygruppe oder Alkanoylreste mit bis zu 4 Kohlenstoffatomen substituiert sein kann, bedeutet, wobei A auch für Salze der 2 oder 3 Stickstoffatome enthaltenden Azole stehen kann,

als herbizidem Wirkstoff und N-Isopropyl-N-propargyl-dichloracetamid als antagonistischem Mittel.

2. Herbizides Mittel nach Anspruch 1, dadurch gekennzeichnet, daß es als herbiziden Wirkstoff 2-Chlor-2',6'-dimethyl-N-(pyrazol-1-yl-methyl)-acetanilid enthält.

3. Herbizides Mittel nach Anspruch 1, dadurch gekennzeichnet, daß das Anteilsverhältnis Acetanilid zu Dichloracetamid 1 : 2 bis 1 : 0,05 Gewichtsteile beträgt.

4. Verfahren zur selektiven Bekämpfung von unerwünschtem Pflanzenwuchs, dadurch gekennzeichnet, daß man herbizide Mittel nach Anspruch 1 vor, bei oder nach der Aussaat der Kulturpflanzen, vor oder während des Auflaufens der Kulturpflanzen ausbringt.

5. Verfahren zur selektiven Bekämpfung von unerwünschtem Pflanzenwuchs, dadurch gekennzeichnet, daß man ein substituiertes Acetanilid der Formel I gemäß Anspruch 1 und N-Isopropyl-N-propargyl-dichloracetamid in einem Verhältnis von 1 : 2 bis 1 : 0,05 Gewichtsteile vor, bei oder nach der Aussat der Kulturpflanzen, vor oder während des Auflaufens der Kulturpflanzen gleichzeitig oder nacheinander in beliebiger Reihenfolge ausbringt.

6. Verfahren zur selektiven Bekämpfung von unerwünschtem Pflanzenwuchs, dadurch gekennzeichnet, daß man das Saatgut der Kulturpflanzen mit N-Isopropyl-N-propargyl-dichloracetamid behandelt und ein substituiertes Acetamid der Formel I gemäß Anspruch 1 vor, bei oder nach der Aussaat der Kulturpflanzen, vor oder während des Auflaufens der Kulturpflanzen ausbringt.

7. Verfahren nach Anspruch 4, 5 und 6, dadurch gekennzeichnet, daß die Kulturpflanze Mais oder Getreide ist.

**Claims**

1. A herbicidal agent characterized by a content of at least one substituted acetanilide of the formula I

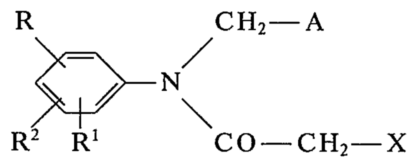

(I)

where R denotes hydrogen, linear or branched alkyl or alkoxy of up to 5 carbon atoms, $R^1$ denotes hydrogen, halogen, or linear or branched alkyl or alkoxy of up to 5 carbon atoms, $R^2$ denotes hydrogen, halogen, or linear or branched alkyl or alkoxy of up to 5 carbon atoms, R together with $R^2$ denotes an alkylene chain of up to 6 carbon atoms which is linked in the o-position and may be substituted by linear or branched alkyl of up to 4 carbon atoms, X denotes chlorine or bromine, and A denotes azole which is attached via a ring nitrogen atom and may be monoor polysubstituted by halogen, phenyl, alkyl, alkoxy, alkylthio or perfluoroalkyl, each of up to 4 carbon atoms, cyano, carboxy, carbalkoxy of up to 4 carbon atoms, or A denotes a salt of an azole containing 2 or 3 nitrogen atoms, as herbicidal active ingredient, and of N-isopropyl-N-propargyl-dichloroacetamide as antagonistic agent.

2. A herbicidal agent as claimed in claim 1, characterized in that it contains 2-chloro-2'6'-dimethyl-N-(pyrazol-1-yl-methyl)-acetanilide as the herbicidal active ingredient.

3. A herbicidal agent as claimed in claim 1, characterized in that the ratio of acetanilide to dichloroacetamide is from 1 : 2 to 1 : 0.05 parts by weight.

4. A process for the selective control of unwanted plant growth, characterized in that a herbicidal agent as claimed in claim 1 is applied before, during or after sowing of the crop plants, or before or during emergence of the crop plants.

5. A process for the selective control of unwanted plant growth, characterized in that a substituted acetanilide of the formula I as claimed in claim 1 and N-isopropyl-N-propargyl-dichloroacetamide in a ratio of from 1 : 2 to 1 : 0.05 parts by weight are applied before, during or after sowing of the crop plants, or before or during emergence of the crop plants, either simultaneously or one after the other in any order.

6. A process for the selective control of unwanted plant growth, characterized in that the seed of the crop plants is treated with N-isopropyl-N-propargyl-dichloroacetamide, and a substituted acetamide of the formula I as claimed in claim 1 is applied before, during or after sowing of the crop plants, or before or during emergence of the crop plants.

7. A process as claimed in claims 4, 5 and 6, characterized in that the crop plant is Indian corn or a cereal.

## Revendications

1. Herbicide caractérisé par le fait qu'il contient, comme principe actif herbicide, au moins un acétanilide substitué de formule I

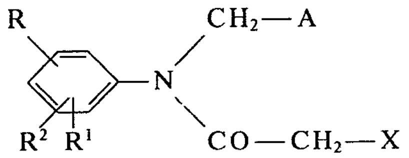

(I)

dans laquelle

R représente l'hydrogène, un reste alkyle ou alcoxy ramifié ou non, ayant jusqu'à 5 atomes de carbone,

$R^1$ l'hydrogène, un halogène, un reste alkyle ou alcoxy, ramifié ou non, à jusqu'à 5 atomes de carbone,

$R^2$ l'hydrogène, un halogène, un reste alkyle ou alcoxy, ramifié ou non, à jusqu'à 5 atomes de carbone,

R avec $R^2$, une chaîne alkylène ayant jusqu'à 6 atomes de carbone, reliée en position ortho, éventuellement substituée par des groupes alkyle, ramifiés ou non, ayant jusqu'à 4 atomes de carbone,

X chlore ou brome et

A un azole lié par l'intermédiaire d'un atome d'azote du noyau, qui peut être substitué une ou plusieurs fois par un halogène, un phényle, un reste alkylalcoxy, alkylthio ou perfluoralkyle, ayant jusqu'à 4 atomes de carbone, cyano, carboxy, carbalcoxy ayant jusqu'à 4 atomes de carbone dans les groupes alcoxy, ou un reste alcanoyle ayant jusqu'à 4 atomes de carbone, A pouvant aussi représenter des sels d'azoles contenant 2 ou 3 atomes d'azote, et, comme agent antagoniste, du N-isopropyl-N-propargyldichloracétamide.

2. Herbicide selon la revendication 1, caractérisé par le fait qu'il contient, comme principe actif herbicide, du 2-chloro-2',6'-diméthyl-N-(pyrazol-1-yl-méthyl)-acétanilide.

3. Herbicide selon la revendication 1, caractérisé par le fait que le rapport, en parties en poids, acétanilide/dichloracétamide est de 1/2 à 1/0,05.

4. Procédé de lutte sélective contre la croissance indésirable de plantes, caractérisé par le fait qu'on applique l'herbicide selon la revendication 1 avant, pendant ou après l'ensemencement des plantes de culture, avant ou pendant la levée des plantes.

5. Procédé de lutte sélective contre la croissance indésirable de plantes, caractérisé par le fait qu'on applique simultanément ou successivement dans un ordre quelconque, avant, pendant ou après l'ensemencement des plantes de culture, avant ou pendant la levée des plantes, un acétanilide de formule I selon la revendication 1 et du N-isopropyl-N-propargyl-dichloracétamide dans un rapport, en parties en poids, de 1/2 à 1/0,05.

6. Procédé de lutte sélective de la croissance indésirable de plantes, caractérisé par le fait qu'on traite la semence des plantes de culture avec du N-isopropyl-N-propargyl-dichloracétamide et on applique un acétanilide substitué de formule I selon la revendication 1 avant, pendant ou après l'ensemencement des plantes de culture, avant ou pendant la levée des plantes de culture.

7. Procédé selon les revendications 4, 5 et 6, caractérisé par le fait que la plante de culture est du maïs ou du blé.